(19) Europäisches Patentamt
European Patent Office
Office européen des brevets

(11) Publication number: **0 098 335**
**B1**

(12) # EUROPEAN PATENT SPECIFICATION

(45) Date of publication of patent specification: **14.01.87**

(51) Int. Cl.$^4$: **C 07 C 31/27**, C 07 C 33/14, C 07 C 29/136, C 08 G 18/32, C 09 D 3/50

(21) Application number: **82303188.5**

(22) Date of filing: **18.06.82**

(54) **Cycloaliphatic alcohols.**

(43) Date of publication of application:
**18.01.84 Bulletin 84/03**

(45) Publication of the grant of the patent:
**14.01.87 Bulletin 87/03**

(84) Designated Contracting States:
**AT BE CH DE FR GB IT LI NL SE**

(56) References cited:
**EP-A-0 017 503**
**US-A-2 415 335**
**US-A-4 038 326**
**US-A-4 348 543**

(73) Proprietor: **HENKEL CORPORATION**
**7900 West 78th Street**
**Minneapolis Minnesota 55435 (US)**

(72) Inventor: **Rogier, Edgar Robert**
**16400 Hidden Valley Road**
**Minnetonka Minnesota 55343 (US)**

(74) Representative: **Cockbain, Julian et al**
**Frank B. Dehn & Co. Imperial House 15-19, Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 098 335 B1

**Description**

This invention relates to polyfunctional alcohols, in particular triols, which may be utilized among other purposes for the preparation of polyurethanes.

It is known that polyfunctional cycloaliphatic acids may be prepared through a Diels-Alder reaction. Such materials are useful in the preparation of polyesters. However, the ester bond itself is subject to attack by a number of chemical agents. It has therefore been found desirable to prepare a compound not subject to an ester attack by eliminating the carboxylic acid functionality. The resultant compounds have been found to possess the advantages of high flexibility when used in urethanes and light stability when the compound is hydrogenated.

A detailed discussion of the art to which the invention relates may be found in United States Patent 4,216,343 (Rogier) dated August 5, 1980 to which attention is particularly drawn. United States Patent 2,415,335 (Bruson) dated February 4, 1947 discloses diols of formulae

$C_4H_9—C_6H_7(CH_2OH)—C_9H_{16}—CH_2OH$ and

$C_4H_9—C_6H_9(CH_2OH)—C_9H_{18}—CH_2OH$

for use in the preparation of linear polyesters and alkyd resins.

According to one aspect of the invention there are provided compounds of formula I

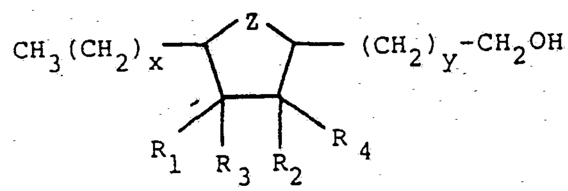

(I)

(wherein $R_1$ and $R_2$, which may be the same or different, represent hydrogen atoms or methyl groups; $R_3$ and $R_4$ each represents a hydroxymethyl group; Z represents a group $—CH=CH—$ or $—CH_2—CH_2—$; x is an integer of from 3 to 6; and y is an integer of from 6 to 9, the sum of x and y being 12; with the proviso that at least one of $R_1$ and $R_2$ represents a hydrogen atom).

As indicated previously, the novel compounds of the present invention may either contain a saturated or unsaturated bond within the six membered ring in their structure. This is particularly important in that should it be desired to promote ultraviolet stability, the bond within the ring structure may be hydrogenated or if a fire retardant property or some further derivatization is desired, the unsaturation may be left in the molecule thereby providing an active site.

The novel polyols of this invention which contain all primary alcohol groups can be prepared by reduction of acids or esters.

According to a further aspect of the invention there is provided a process for the preparation of compounds of formula I which process comprises reducing a compound of formula II

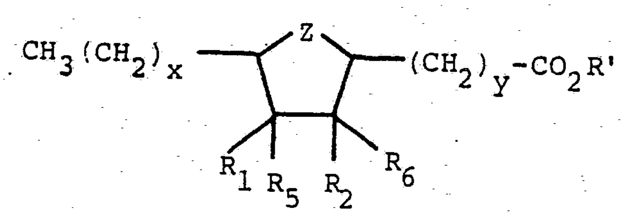

(II)

(wherein $R_1$, $R_2$, x, y and Z are as defined above; $R_5$ and $R_6$ each represents a group of formula $—CO_2R'$; and each R'; which may be the same or different, represents a hydrogen atom or an alkyl group; with the proviso that if Z represents a group $—CH=CH—$ then either or both aliphatic chains $CH_3(CH_2)_x—$ and $—(CH_2)_y—$ may be unsaturated).

In the acid or ester of formula II, x is preferably 4 or 5, y is preferably 7 or 8, and R', if it represents an alkyl group, preferably contains from 1 to 6 carbon atoms. Further unsaturation in the aliphatic side chains of formula II may be present if a material such as tung oil is employed.

The carbon-carbon double bonds of the above cyclohexane derivatives of formula II wherein Z represents a $—CH=CH—$ group can be saturated by catalytic hydrogenation using catalysts such as Raney nickel, supported nickels, palladium or platinum.

Either unsaturated or saturated esters of formula II can be converted into saturated polyols of formula I by hydrogenolysis using copper chromite catalysts such as those described by Adkins J. Amer. Chem. Soc. 54, 1138 (1932) or commercially available modifications of copper chromite catalysts.

If the unsaturated polyols of formula I (wherein Z represents a $—CH=CH—$ group) are desired, the ester groups of the unsaturated esters of formula II' (wherein Z represents a $—CH=CH—$ group) can be hydrogenolyzed with the carbon-carbon double bonds being preserved by using chemical reducing agents such as alkali metal alcoholates (e.g. sodium metal and alcohols — Bouveault-Blanc reductions) or $LiAlH_4$.

The acid and ester reagents of formula II may be prepared from a variety of conjugated fatty acids or their derivatives and a dieneophile, suitably by a Diels-Alder reaction as previously described. Basically, the Diels-Alder reaction is conducted after obtaining the methyl ester of a drying or semi-drying oil through methanolysis. Conveniently, the methyl ester may be obtained by methanolysis of tung oil. For the sake of simplicity, methanolysis is mentioned, however, any alcoholysis reaction may be employed.

Variations in the present invention allow the use of various unsaturated materials which are derived from the semi-drying oils such as those selected from the group consisting of soybean oil, tall oil, tung oil, linseed oil, safflower oil, sunflower oil and other similar materials.

Various unsaturated acids may be utilized herein as previously indicated including those conveniently having 18 carbon atoms such as 9,12-octadecadienoic acid (linoleic acid); 9,11-octadecadienoic acid; 10,12-octadecadienoic acid; 9,12,15-octadecatrienoic acid (linolenic acid); 6,9,12-octadecatrienoic acid; 9,11,13-octadecatrienoic acid (eleostearic acid); 10,12,14-octadecatrienoic acid and other similar materials. It is also noted as previously stated that various derivatives of the fatty acids may be employed, particularly the alkyl esters and particularly those containing up to 8 carbon atoms. Most conveniently, however, the methyl ester is employed for reasons of convenience and expense. It is also noted that a particularly interesting variable in the present invention is to conjugate the double bonds of the fatty acids described above and such may be done by known techniques such as alkali conjugation. Conjugation by some method is necessary for the Diels-Alder reaction.

Dieneophiles which can be used in the preparation of compounds of formula II wherein Z represents a —CH=CH— group include acrylic acid, methacrylic acid, crotonic acid, maleic acid, fumaric acid and the $C_1$ to $C_8$ esters of such acids. Maleic anhydride may also be employed.

The dieneophile and methyl ester of the drying oil together with a polymerization inhibitor such as para-methoxyphenol are charged into a reactor flushed with nitrogen and heated to a temperature of from about 110 to 180 degrees C for a substantial period of time. After the reaction is complete, any excess dieneophile is then removed and the unpurified unsaturated cycloaliphatic material is obtained.

Wherein the saturated material is required, the above unsaturated product of formula II can be reduced using a common hydrogenation catalyst. Most practically platinum and palladium on carbon can be utilized. The material as described above is then saturated by placing it in a reaction vessel with the 5 percent palladium on carbon catalyst and the reaction vessel is flushed with nitrogen followed by pressurization with hydrogen gas at from about 1 to about 500 atmospheres (0.1 to 50.7 $MNm^{-2}$). The temperature is maintained during the hydrogenation at from about 20 to 150, preferably 55 to 135, degrees Celsius. The pressure during the hydrogenation reaction is conveniently maintained at from about 10 to about 200 atmospheres (1.0 to 20.3 $MNm^{-2}$). The reaction time is simply that required to obtain as high a degree of hydrogenation as is desired. Conveniently, this reaction may be conducted for from about 3 to 8 hours.

Thereafter, the acid or ester may be reacted utilizing hydrogen gas and copper chromite utilizing conditions for reduction of the acid or ester to the corresponding alcohols of formula I.

It has been found that such a hydrogenolysis reaction with the copper chromite catalyst may be conducted at from about 240 to about 300 degrees C, preferably from about 250 to about 280 degrees C. The pressure for the hydrogenation reaction may be maintained at from about 200 to 500 atmospheres (20.3 to 50.7 $MNm^{-2}$), preferably from about 220 to about 480 atmospheres (22.3 to 48.6 $MNm^{-2}$).

The compounds of the present invention may be used to prepare urethanes and melamine coatings.

According to a further aspect of the present invention there is provided the use of compounds of formula I in the preparation of urethane or melamine coatings or mouldings.

The following Examples are provided to illustrate the scope of the present invention.

Throughout this specification, percentages and ratios are by weight and temperatures in degrees Celsius unless otherwise indicated.

### Example I (Preparative)
### Preparation of Triester

Into a 5 litre Pyrex flask equipped with a reflux condenser and source of nitrogen purge is placed 1208 grams of the methyl esters of tung oil fatty acids (77 percent eleostearic acid) and 636 grams of dimethyl maleate. The reaction system is flushed with nitrogen and heated at 180 degrees C for 24 hours.

The reaction product is combined with the product of a similar run starting with 301 grams of methyl tungate. The combined crude products after stripping of excess dimethyl maleate by distillation under reduced pressure weigh 2222 grams. Thin-film distillation of 2191 grams of this residue yielded the following fractions:

| Fraction No. | Pressure in torr (figures in brackets in $Nm^{-2}$ | Weight (g) | Saponification equivalent weight | % Triester* of formula IV |
|---|---|---|---|---|
| 1 | 0.8—0.2 (107 to 27) | 499 | | |
| 2 | 0.4 (53) | 694 | 181 | 98 |
| 3 | 0.2—0.4 (27 to 53) | 600 | 172 | 88 |
| 4 | 0.4 (53) | 92 | | 44 |
| Residue | | | 124 | |
| Trap condensate | | 123 | | |

* Determined by Gas Chromatography. Sum of all isomer triesters.

The triester product (methyl 10-(3-n-butyl-1,2-di-methoxycarbonyl-cyclohex-5-enyl)-dec-9-enoate) is of formula IV

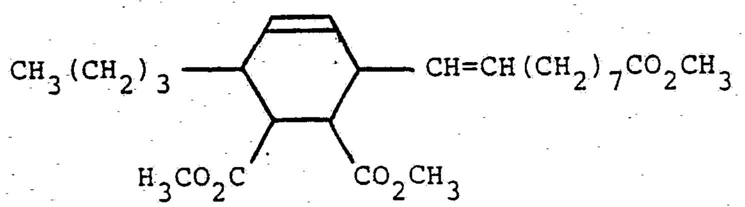

(IV)

Example II
Hydrogenolysis of Triester

Into a litre, 316 SS Magne-Drive autoclave is charged 497 grams of the triester product of Example I of formula IV (Fraction 2) and 50 grams of copper chromite catalyst (Cu—1910—P, Harshaw Chemical Co.). The autoclave is flushed with nitrogen, then charged with hydrogen to 200 atmosphere (20.3 $MNm^{-2}$) and heated to 270 degrees C. The reaction is maintained at 270 degrees C and 200—250 atmospheres (20.3 to 25.3 $MNm^{-2}$) pressure for 12 hours. The reaction is cooled, vented, flushed with nitrogen and filtered under pressure. The filtered product weighed 315 grams after vacuum stripping and had a hydroxyl equivalent weight of 286. The product was essentially the triol of formula V

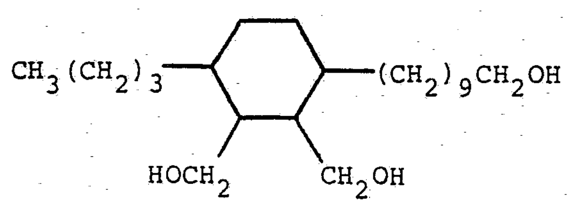

(V)

10-(3-n-butyl-1,2-dihydroxymethyl-cyclohexyl)-decan-1-ol.

The polyols of formula I herein are suitable for melamine curing agents, for urethane preparation and similar uses requiring hydroxyl functionality or hydroxyl with unsaturation. The esters of formula II are, of course, useful as the precursors to the various polyols.

**Claims for the Contracting States: BE CH DE FR GB IT LI NL SE**

1. Compounds of the formula I

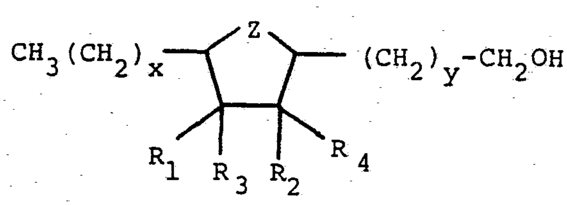

(I)

(wherein $R_1$ and $R_2$, which may be the same or different, represent hydrogen atoms or methyl groups; $R_3$ and $R_4$ each represent a hydroxymethyl group; Z represents a group —CH=CH— or —$CH_2$—$CH_2$—; x is an integer of from 3 to 6; and y is an integer of from 6 to 9, the sum of x and y being 12; with the proviso that at least one of $R_1$ and $R_2$ represents a hydrogen atom).

2. Compounds as claimed in claim 1 wherein $R_1$ and $R_2$ both represent hydrogen atoms.

3. Compounds as claimed in either of claims 1 and 2 wherein x is 4 or 5 and y is 7 or 8.

4. 10-(3-n-butyl-1,2-di-hydroxymethyl-cyclohexyl)-decan-1-ol, being a compound as claimed in claim 1.

5. A process for the preparation of compounds of formula I (as defined in claim 1) which process comprises reducing a compound of formula II

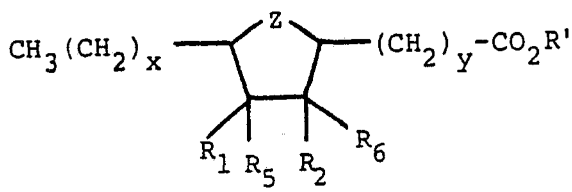

(II)

(wherein $R_1$, $R_2$, x, y and Z are as defined in claim 1; $R_5$ and $R_6$ each represents a group of formula —$CO_2R'$; and each R', which may be the same or different, represents a hydrogen atom or an alkyl group; with the proviso that if Z represents a group —CH=CH— then either or both aliphatic chains $CH_3(CH_2)_x$— and —$(CH_2)_y$— may be unsaturated).

6. A process as claimed in claim 5 wherein the reduction of the compound of formula II is effected by catalysed hydrogenolysis or by the use of alkali metal alcoholates or lithium aluminium hydride.

7. The use of compounds of formula I (as defined in claim 1) in the preparation of urethane or melamine coatings or mouldings.

**Claims for the Contracting State: AT**

1. A process for the preparation of compounds of formula I

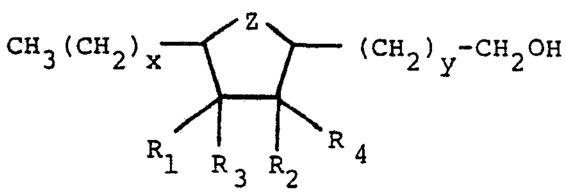

(I)

(wherein $R_1$ and $R_2$, which may be the same or different, represent hydrogen atoms or methyl groups; $R_3$ and $R_4$ each represent a hydroxymethyl group; Z represents a group —CH=CH— or —$CH_2$—$CH_2$—; x is an integer of from 3 to 6; and y is an integer of from 6 to 9, the sum of x and y being 12; with the proviso that at least one of $R_1$ and $R_2$ represents a hydrogen atom) which process comprises reducing a compound of formula II

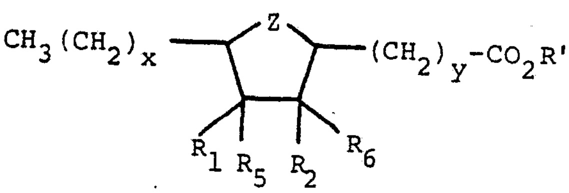

(II)

(wherein $R_1$, $R_2$, x, y and Z are as defined above; $R_5$ and $R_6$ each represents a group of formula —$CO_2R'$; and each R', which may be the same of different, represents a hydrogen atom or an alkyl group; with the proviso that if Z represents a group —CH=CH— then either or both aliphatic chains $CH_3(CH_2)_x$— and —$(CH_2)_y$— may be unsaturated).

2. A process as claimed in claim 1 wherein a compound of formula II in which Z represents a —CH=CH— group is reduced to a compound of formula I in which Z represents a —$CH_2$—$CH_2$— group by a reaction involving catalytic hydrogenation.

3. A process as claimed in claim 1 wherein a compound of formula II is reduced by catalytic hydrogenolysis.

4. A process as claimed in claim 1 wherein a compound of formula II in which Z represents a —CH=CH— group is reduced by alkali metal alcoholates or lithium aluminium hydride to a compound of

formula I in which Z also represents a —CH=CH— group.

5. A process as claimed in any one of the preceding claims wherein in the compound of formula II $R_1$ and $R_2$ both represent hydrogen atoms.

6. A process as claimed in any one of the preceding claims wherein in the compound of formula II x is 4 or 5 and y is 7 or 8.

7. A process as claimed in claim 1 wherein the compound of formula II is methyl 10-(3-n-butyl-2,3-dimethoxycarbonyl-cyclohex-5-enyl)-dec-9-enoate.

8. The use of compounds of formula I (as defined in claim 1) in the preparation of urethane or melamine coatings or mouldings.

**Patentansprüche für die Vertragsstaaten: BE CH DE FR GB IT LI NL SE**

1. Verbindungen der Formel I

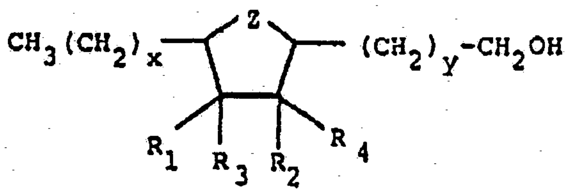

(I)

(in der

$R_1$ und $R_2$, die gleich oder verschieden sein können, Wasserstoff-Atome oder Methyl-Gruppen bezeichnen,

$R_3$ und $R_4$ jeweils eine Hydroxymethyl-Gruppe bezeichnen,

Z eine Gruppe —CH=CH— oder —$CH_2$—$CH_2$— bezeichnet,

x eine ganze Zahl von 3 bis 6 ist und

y eine ganze Zahl von 6 bis 9 ist, wobei die Summe von x und y 12 ist,

mit der Maßgabe, daß wenigstens einer der Substituenten $R_1$ und $R_2$ ein Wasserstoff-Atom bezeichnet).

2. Verbindungen nach Anspruch 1, dadurch gekennzeichnet, daß $R_1$ und $R_2$ beide Wasserstoff-Atome bezeichnen.

3. Verbindungen nach Ansprüchen 1 oder 2, dadurch gekennzeichnet, daß x 4 oder 5 ist und y 7 oder ist.

4. 10-(3-n-Butyl-1,2-di-hydroxymethyl-cyclohexyl)decan-1-ol, das eine Verbindung nach Anspruch 1 ist.

5. Verfahren zur Herstellung von Verbindungen der Formel I (wie in Anspruch 1 definiert) durch Reduzieren einer Verbindung der Formel II

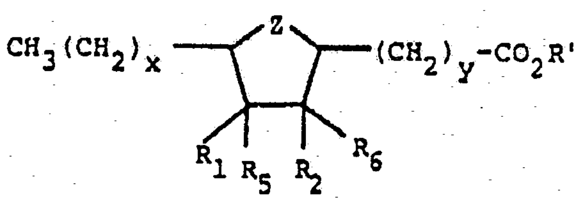

(II)

(in der

$R_1$, $R_2$, x, y und Z die in Anspruch 1 angegebenen Bedeutungen haben,

$R_5$ und $R_6$ jeweils eine Gruppe der Formel —$CO_2R'$ bezeichnen und jedes der

R', die jeweils gleich oder verschieden sein können, ein Wasserstoff-Atom oder eine Alkyl-Gruppe bezeichnet,

mit der Maßgabe, daß, wenn Z eine Gruppe —CH=CH— bezeichnet, entweder eine oder beide aliphatischen Ketten $CH_3(CH_2)_x$— und —$(CH_2)_y$— ungesättigt sein können).

6. Verfahren nach Anspruch 5, dadurch gekennzeichnet, daß die Reduktion der Verbindung der Formel II durch katalytische Hydrogenolyse oder durch die Verwendung von Alkalimetallalkoholaten oder Lithiumaluminiumhydrid erfolgt.

7. Verwendung von Verbindungen der Formel I (wie in Anspruch 1 definiert) bei der Herstellung von Urethan- oder Melamin-Beschichtungen oder Formteilen.

**Patentansprüche für den Vertragsstaat: AT**

1. Verfahren zur Herstellung von Verbindungen der Formel I

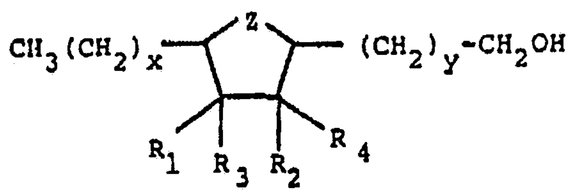

(I)

(in der

$R_1$ und $R_2$, die gleich oder verschieden sein können, Wasserstoff-Atome oder Methyl-Gruppen bezeichnen,

$R_3$ und $R_4$ jeweils eine Hydroxymethyl-Gruppe bezeichnen,

Z eine Gruppe —CH=CH— oder —$CH_2$—$CH_2$— bezeichnet,

x eine ganze Zahl von 3 bis 6 ist und

y eine ganze Zahl von 6 bis 9 ist, wobei die Summe von x und y 12 ist,

mit der Maßgabe, daß wenigstens einer der Substituenten $R_1$ und $R_2$ ein Wasserstoff-Atom bezeichnet), durch Reduzieren einer Verbindung der Formel II

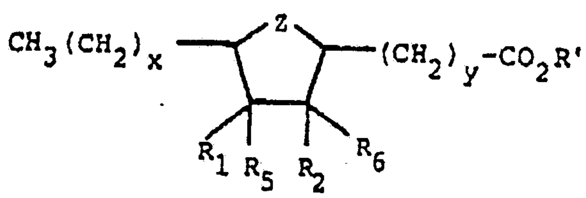

(II)

(in der

$R_1$, $R_2$, x, y und Z die oben angegebenen Bedeutungen haben,

$R_5$ und $R_6$ jeweils eine Gruppe der Formel —$CO_2R'$ bezeichnen und jedes der

R', die jeweils gleich oder verschieden sein können, ein Wasserstoff-Atom oder eine Alkyl-Gruppe bezeichnet,

mit der Maßgabe, daß, wenn Z eine Gruppe —CH=CH— bezeichnet, entweder eine oder beide aliphatischen Ketten $CH_3(CH_2)_x$— und —$(CH_2)_y$— ungesättigt sein können).

2. Verfahren nach Anspruch 1, dadurch gekennzeichnet, daß eine Verbindung der Formel II, in der Z eine Gruppe —CH=CH— bezeichnet, durch eine Reaktion unter katalytischer Hydrierung zu einer Verbindung der Formel I, in der Z eine Gruppe —$CH_2$—$CH_2$— bezeichnet, reduziert wird.

3. Verfahren nach Anspruch 1, dadurch gekennzeichnet, daß eine Verbindung der Formel II durch katalytische Hydrogenolyse reduziert wird.

4. Verfahren nach Anspruch 1, dadurch gekennzeichnet, daß eine Verbindung der Formel II, in der Z eine Gruppe —CH=CH— bezeichnet, durch Alkalimetallalkoholate oder Lithiumaluminiumhydrid zu einer Verbindung der Formel I, in der Z ebenfalls eine Gruppe —CH=CH— bezeichnet, reduziert wird.

5. Verfahren nach irgendeinem der vorhergehenden Ansprüche, dadurch gekennzeichnet, daß in der Verbindung der Formel II $R_1$ und $R_2$ beide Wasserstoff-Atome bezeichnen.

6. Verfahren nach irgendeinem der vorhergehenden Ansprüche, dadurch gekennzeichnet, daß in der Verbindung der Formel II x 4 oder 5 ist und y 7 oder 8 ist.

7. Verfahren nach Anspruch 1, dadurch gekennzeichnet, daß eine Verbindung der Formel II 10-(3-n-Butyl-2,3-di-methoxycarbonyl-cyclohex-5-enyl)dec-9-enoat ist.

8. Verwendung von Verbindungen der Formel I (wie in Anspruch 1 definiert) bei der Herstellung von Urethan- oder Melamin-Beschichtungen oder Formteilen.

**Revendications pour les Etats contractants: BE CH DE FR GB IT LI NL SE**

1. Composés de formule I

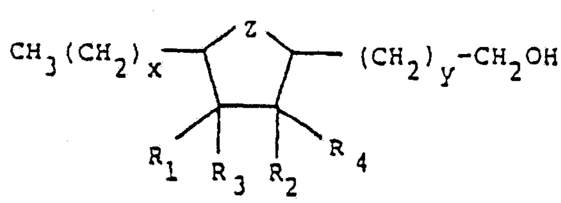

(I)

(dans laquelle $R_1$ et $R_2$, qui peuvent être identiques ou différents, représentent des atomes d'hydrogène ou des groupes méthyle; $R_3$ et $R_4$ représentent chacun un groupe hydroxyméthyle; Z représente un groupe $—CH{=}CH—$ ou $—CH_2—CH_2—$; x est un nombre entier valant de 3 à 6; et y est un nombre entier valant de 6 à 9, la somme de x et de y valant 12; à la condition qu'au moins l'un des symboles $R_1$ et $R_2$ représente un atome d'hydrogène).

2. Composés tels que revendiqués dans la revendication 1, dans lesquels $R_1$ et $R_2$ représentent tous deux des atomes d'hydrogène.

3. Composés tels que revendiqués dans l'une des revendications 1 et 2, dans lesquels x vaut 4 ou 5 et 7 vaut 7 ou 8.

4. Le 10-(3-n-butyl-1,2-di-hydroxyméthyl-cyclohexyl)-décane-1-ol, qui est un composé tel que revendiqué dans la revendication 1.

5. Procédé pour préparer des composés de formule I (telle que définie à la revendication 1), ce procédé comprenant la réduction d'un composé de formule II

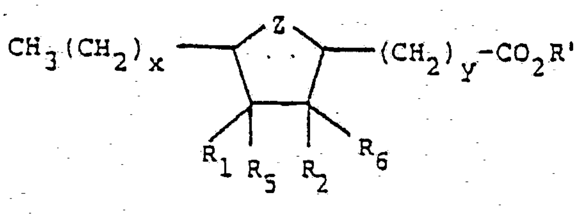

(II)

(dans laquelle $R_1$, $R_2$, x, y et z sont tels que définis à la revendication 1; $R_5$ et $R_6$ représentent chacun un groupe de formule $—CO_2R'$; et les symboles R', qui peuvent être identiques ou différents, représentent chacun un atome d'hydrogène ou un groupe alkyle; à la condition que, si Z représente un groupe $—CH{=}CH—$, l'une des chaînes aliphatiques $CH_3(CH_2)_x—$ et $—(CH_2)_y—$, ou les deux, peut ou peuvent etre insaturée(s)).

6. Procédé tel que revendiqué à la revendication 5, dans lequel on effectue la réduction du composé de formule II par hydrogénolyse catalysée ou par l'utilisation d'alcoolates de métaux alcalins ou d'hydrure de lithium et d'aluminium.

7. Utilisation de composés de formule I (tels que définis à la revendication 1) dans la préparation de revêtements ou de pièces moulées en uréthanne ou mélamine.

**Revendications pour l'Etat contractant: AT**

1. Procédé pour la préparation de composés de formule I

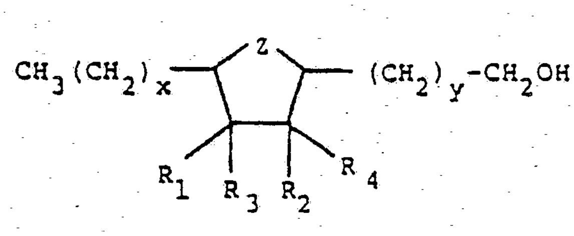

(I)

(dans laquelle $R_1$ et $R_2$, qui peuvent être identiques ou différents, représentent des atomes d'hydrogène ou des groupes méthyle; $R_3$ et $R_4$ représentent chacun un groupe hydroxyméthyle; Z représente un groupe $—CH{=}CH—$ ou $—CH_2—CH_2—$; x est un nombre entier valant de 3 à 6; et y est un nombre entier valant de 6 à 9, la somme de x et de y valant 12; à la condition qu'au moins l'un des symboles $R_1$ et $R_2$ représente un atome d'hydrogène), ce procédé comprenant la réduction d'un composé de formule II

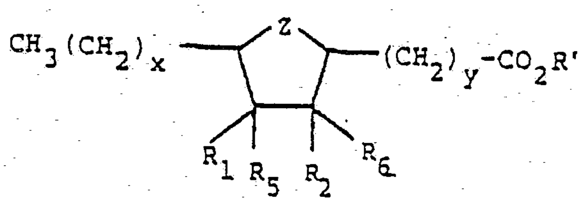

(II)

(dans laquelle $R_1$, $R_2$, x, y et z sont tels que définis ci-dessus; $R_5$ et $R_6$ représentent chacun un groupe de formule $—CO_2R'$; et les symboles R', qui peuvent être identiques ou différents, représentent chacun un atome d'hydrogène ou un groupe alkyle; à la condition que, si Z resente un groupe $—CH{=}CH—$, l'une des chaînes aliphatiques $CH_3(CH_2)_x—$ et $—(CH_2)_y$, ou les deux, peut ou peuvent être insaturée(s)).

2. Procédé tel que revendiqué à la revendication 1, dans lequel on réduit un composé de formule II (dans laquelle Z représente un groupe —CH=CH—) en un composé de formule I (dans laquelle Z représente un groupe —$CH_2$—$CH_2$—) par une réaction impliquant une hydrogénation catalytique.

3. Procédé tel que revendiqué à la revendication 1, dans lequel on réduit par hydrogénolyse catalytique un composé de formule II.

4. Procédé tel que revendiqué à la revendication 1, dans lequel on réduit un composé de formule II, dans laquelle Z représente un groupe —CH=CH—, par des alcoolates de métaux alcalins ou de l'hydrure de lithium et d'aluminium en un composé de formule I, dans laquelle Z représente aussi un groupe —CH=CH—.

5. Procédé tel que revendiqué dans l'une quelconque des revendications précédentes, dans lequel, dans le composé de formule II, $R_1$ et $R_2$ représentent tous deux des atomes d'hydrogène.

6. Procédé tel que revendiqué dans l'une quelconque des revendications précédentes dans lequel, dans le composé de formule II, x vaut 4 ou 5 et y vaut 7 ou 8.

7. Procédé tel que revendiqué à la revendication 1, dans lequel le composé de formule II est le 10-(3-n-butyl-2,3-di-méthoxycarbonylcyclohex-5-ényl)-déc-9-énoate de méthyle.

8. Utilisation de composés de formule I (tels que définis à la revendication 1) dans la préparation de revêtements ou de pièces moulées en uréthanne ou en mélamine.